# EUROPÄISCHE PATENTSCHRIFT

(11) **EP 1 114 619 B1**
(45) Veröffentlichungstag und Bekanntmachung des Hinweises auf die Patenterteilung: **14.12.2005**
(21) Anmeldenummer: 99126168.6
(22) Anmeldetag: 29.12.1999
(51) Int. Cl.: A61B 17/64, A61F 5/01, A63B 23/04

(54) **Vorrichtung zur Ausübung einer statischen oder dynamischen vorderen, resp. hinteren Translationskraft am Kniegelenk**
Device for static or dynamic application of anterior, resp. posterior translational force on the knee joint
Dispositif pour l'application au genou d'une force de translation statique ou dynamique anterieure, resp. posterieure

(43) Veröffentlichungstag der Anmeldung: 11.07.2001
(73) Patentinhaber: Jakob, Roland Peter, 1787 Môtier (CH); Gautier, Emanuel, 1700 Fribourg (CH)
(72) Erfinder: Jakob, Roland Peter, 1787 Môtier (CH); Gautier, Emanuel, 1700 Fribourg (CH)
(74) Vertreter: Bauer, Friedrich

(56) Entgegenhaltungen:
- US-A- 4 865 024
- US-A- 5 036 837
- US-A- 5 547 464
- US-A- 5 624 389
- US-A- 5 662 595
- US-A- 5 810 752

## Beschreibung

Die Erfindung betrifft eine Vorrichtung zur Ausübung einer Translationskraft auf die Tibia für die Behandlung oder Nachbehandlung der hinteren oder vorderen Kreuzbandinstabilität und assoziierter peripherer Knieinstabilitäten, welche einen Lastübertragungsarm und zwei im Bereich des Kniegelenks befestigbare Teile aufweist, die über den Lastübertragungsarm ohne starre femorotibiale Verbindung miteinander wirkverbunden sind, um die Translationskraft vom Femur auf die Tibia zu übertragen, gemäß dem Oberbegriff der Ansprüche 1 und 2.

Die Behandlung der frischen oder chronischen hinteren Kreuzbandruptur sowie assoziierter Läsionen wie posterolaterale, laterale, sowie mediale Seitenbandruptur, aber theoretisch auch der vorderen Kreuzbandinstabilität stellen besondere Anforderungen. Bisherige äussere Stützapparate (Braces), die mit einem gelenkigen Apparat Stabilität bewirken sollten, haben keine befriedigenden Ergebnisse erbracht. Sie sind deshalb weitgehend aus der routinemässigen Anwendung verschwunden. Der Grund dafür liegt darin, dass sie nicht im Stande sind, eine dynamische Kraft auszuüben oder aufrecht zu erhalten und gleichzeitig die Beweglichkeit des Kniegelenkes, wenn auch limitiert, zu gewährleisten.

Einige dynamische Schienen für die Behandlung der medialen oder lateralen Instabilität existieren auf dem Markt und sind eingesetzt einerseits zur Behandlung einer Instabilität oder einer unikompartimentären Gonarthrose (Monarchschiene). Sie entlasten beispielsweise das mediale Kompartiment bei gleichzeitiger O-Bein-Fehlstellung, indem sie eine X-Bein-Kraft (Valguskraft) ausüben.

Die Behandlung und v.a. Nachbehandlung der *hinteren Kreuzbandläsion* ist bis heute nicht gelöst, obwohl die chirurgischen Rekonstruktionstechniken recht weit entwickelt sind. Unter der andauernden nach dorsal gerichteten Schwerkraft (beim Sitzen, beim Liegen auf dem Rücken) dehnt sich das gerissene und konservativ behandelte, chirurgisch genähte oder rekonstruierte Band in den ersten zwei- bis fünf Monaten nach der Operation, so dass von der initial guten Stabilität mindestens 50 % wieder verloren gehen. Die Frage stellt sich, ob sich der operative Aufwand lohnt, wenn während der frühen und mittelfristigen Rehabilitationsphase eine sekundäre Bandinsuffizienz auftritt, welche durch eine ungünstige Krafteinwirkung entsteht.

Die *chronische vordere Instabilität* ist bezüglich der Ergebnisse operativer Behandlung insgesamt als günstiger zu bewerten, wahrscheinlich weil die Tibia grundsätzlich in einer reponierten Position steht. Es gibt allerdings auch hier Situationen, die als ungünstige Ausgangsposition zu bewerten sind :
- ausgeprägte vordere Instabilität mit massiver Auslockerung der sekundären, peripheren widerhaltenden Bandzügel
- gleichzeitige Vorhandensein einer ausgeprägten Neigung des Tibiaplateau nach dorsal.

Die dorsale Neigung des Tibiaplateaus misst normalerweise zwischen 4-6°. Nimmt diese Neigung auf 10° oder mehr zu, was entweder konstitutionell, d.h. anlagebedingt sein kann, oder was durch eine chronische vordere Kreuzbandinstabilität entstehen kann, so bildet sich bei Belastung ein im Stehen wirksamer, nach vorne gerichteter Kraftvektor für die Tibia aus. Dies bewirkt, dass das Femur auf der schrägen Tibiagelenkfläche nach hinten, resp. die Tibia nach vorne gedrängt wird. Dabei wird das vordere Translationsphänomen der Tibia durch eine gleichzeitige vordere Kreuzbandinsuffizienz verstärkt. Ein in dieser Situation rekonstruiertes vorderes Kreuzband wird somit massiv mechanisch beansprucht und in der Phase des biologischen Remodelling während der Revaskularisationsphase irreversibel gedehnt. Dies ist auch der Grund dafür, dass eine solche Kreuzbandplastik nicht lohnenswert ist, und der Patient am Ende der Behandlung eine identische Knieinstabilität aufweist wie vor der Operation

Aus der US 5 810 752 ist eine Vorrichtung gemäß dem Oberbegriff der Ansprüche 1 und 2 bekannt, bei der eine Oberschenkelmanschette mit einer Unterschenkelmanschette über beidseits des Knies verlaufende, stabartige Schienen miteinander verbunden sind. Jede Schiene besteht aus zwei Schienenteilen, die sich teleskopartig zusammenschieben bzw. auseinanderziehen lassen, so dass dort keine starre femorotibiale Verbindung vorhanden ist. Zwischen den Schienen erstreckt sich ein Zugband, das über die Vorderseite des Unterschenkels in der Nähe des Schienbeinkopfs geführt ist, wodurch auf die Tibia im knienahen Bereich eine statische Translationskraft aufgebracht wird, die nach hinten, d.h. in dorsaler Richtung, wirkt.

Aus der US 5 547 464 ist eine Orthese bekannt, bei der die Oberschenkel- und Unterschenkelschienen teleskopartig verschiebbar in zugeordneten Gelenkschienen geführt sind, die wiederum mittels eines Gelenks schwenkbar miteinander verbunden sind. Mit dieser bekannten Vorrichtung lassen sich jedoch keine oder keine ausreichenden nach vorne oder hinten gerichtete Translationskräfte auf die Tibia im Bereich des Knies aufbringen.

Aus der US 5 624 389 ist eine Kniegelenksorthese bekannt, bei der die Oberschenkelschiene schwenkbar an der Unterschenkelschiene befestigt ist. Weiterhin ist dort ein Federkraftmechanismus vorgesehen, der bewirkt, dass das Schwenken der Schiene bis zu einem Schwenkwinkel von etwa 30 bis 40° nur gegen eine zunehmende Schwenkkraft möglich ist, während beim weiteren Schwenken der Schiene in Flexionsrichtung die aufzubringende Schwenkkraft wieder abnimmt.

Der Erfindung liegt die Aufgabe zugrunde, eine Vorrichtung der eingangs genannten Art zu schaffen, mit der die Behandlung oder Nachbehandlung der hinteren oder vorderen Kreuzbandinstabilitäten und assoziierter peripherer Knieinstabilitäten auf besonders wirkungsvolle Weise durchgeführt werden kann.

Diese Aufgabe wird erfindungsgemäß durch die Merkmale der Ansprüche 1 und 2 gelöst. Vorteilhafte Ausführungsformen der Erfindung sind in den weiteren Ansprüchen beschrieben.

Bei der erfindungsgemäßen Vorrichtung gemäß Anspruch 1 besteht eines der im Bereich des Kniegelenks befestigbaren Teile aus einem Fixateur externe, welcher am Femur fixierbar ist und ein eine statische oder dynamische Translationskraft erzeugendes Kraftelement trägt. Weiterhin umfasst das andere Teil ein mit Rollen oder Gleitmechanismen versehenes, quer in die Tibia einbringbares mechanisches Element oder mit Rollen oder Gleitmechanismen versehene, seitlich an einer Unterschenkelorthese befestigbaren Bolzen. Der Lastübertragungsarm steht ohne starre Verbindung mit dem mechanischen Element oder den Bolzen über die am Element bzw. den Bolzen angeordneten Rollen oder Gleitmechanismen in Wirkverbindung, um die Translationskraft nach vorne oder hinten auf die Tibia zu übertragen.

Bei der erfindungsgemäßen Vorrichtung gemäß Anspruch 2 ist an einer Oberschenkelorthese ein eine statische oder dynamische Translationskraft erzeugendes Kraftelement befestigt. Weiterhin umfasst das andere Teil ein mit Rollen oder Gleitmechanismen versehenes, quer in die Tibia einbringbares mechanisches Element oder mit Rollen oder Gleitmechanismen versehene, seitlich an einer Unterschenkelorthese befestigbaren Bolzen. Der Lastübertragungsarm steht ohne starre Verbindung mit dem mechanischen Element oder den Bolzen über die am Element bzw. den Bolzen angeordneten Rollen oder Gleitmechanismen in Wirkverbindung, um die Translationskraft nach vorne oder hinten auf die Tibia zu übertragen.

Die vorliegende Erfindung beruht somit auf der Verwendung eines äußeren Apparates zur alleinig konservativen Behandlung oder zur Nachbehandlung operativ rekonstruierter oder genähter vorderer oder hinterer Kreuzbandläsionen. Der Apparat übt eine statische oder dynamische, in ihrer Größe einstellbare, nach vorne oder nach hinten gerichtete Translationskraft, übertragen vom Femur auf die Tibia, aus. Diese Kraft wird durch mindestens ein Kraftelement, vorzugsweise durch ein Federsystem, generiert und entweder über ein modifiziertes Fixateur externe System oder über entsprechend an den Ober- oder Unterschenkel angeformte Orthesen vom Femur auf die Tibia übertragen. Dabei wird der natürliche Roll-Gleitmechanismus des Femurkondylus gegenüber dem Tibiaplateau bei Flexions-Extensionsbewegungen des Kniegelenkes nicht kompromittiert. Wesentlich ist hierbei, dass keine apparative Verbindung im Sinne einer durch den Apparat vorgegebenen Rotationsachse zwischen Oberschenkel und Unterschenkel besteht.

Der Effekt dieser Vorrichtung liegt in der Möglichkeit, die Tibia im Kniegelenk statisch oder dynamisch nach ventral, resp. dorsal vorzuspannen und nach vorne, resp. hinten zu bewegen, sei es zu ziehen oder zu drücken, und dadurch das genähte oder rekonstruierte hintere, resp. vordere Kreuzband sowie begleitende posteromediale und posterolaterale, resp. anteromediale und anterolaterale rupturierte oder gedehnte, genähte oder rekonstruierte Strukturen in der frühen und mittelfristigen Heilungsphase zu entlasten und dadurch zu schützen. Später, nach Abschluss der Revaskularisation und des Remodellings des kollagenen Gewebes hat dieses eine genügende mechanische Resistenz entwickelt, so dass die Vorrichtung entfernt werden kann ohne Gefahr zu laufen, dass die Ausgangsposition in hinterer, resp. vorderer Schublade mit entsprechender subjektiver und objektiver Knieinstabilität wieder eingenommen wird.

Der Unterschied zwischen den skelettären, direkt am Knochen angreifenden und den via "Brace" applizierten Kräften liegt eindeutig in der direkteren und effizienteren, skelettären Krafteinwirkung. Bei der via Brace applizierten Kraft besteht eine gewisse Kompression und Deformation der Weichteile und somit auch der neurovaskulären Strukturen, was beim gefäßgesunden Patienten keinen Nachteil darstellt, unmittelbar postoperativ jedoch wegen Kompromittierung des venösen Abflusses problematisch sein könnte.

Zweckmäßigerweise ist der Fixateur externe mit mindestens zwei Schrauben in der Form von Schanzschrauben am Femur fixierbar.

Zweckmäßigerweise besteht das in die Tibia eingebrachte mechanische Element aus einem Steinmannnagel.

Der Lastübertragungsarm kann einseitig oder beidseitig des Kniegelenks herabgeführt sein.

Zweckmäßigerweise ist das mindestens eine Kraftelement in einer oder mehreren Ebenen starr fixierbar.

Das Kraftelement kann eine Spiralfeder, welche in Richtung ihrer Längsachse auf Zug oder Druck oder quer zu ihrer Längsachse beansprucht ist, ein System von Blattfedern, eine Uhrenfeder, einen Torsionsstab, ein pneumatisches, hydraulisches, osmotisches, elektromagnetisches, magnetisches, Bimetall- oder Memory-Element umfassen.

Zweckmäßigerweise ist die Translationskraft in Streckstellung in ihrer Größe einstellbar.

Die Erfindung wird nachfolgend anhand der Zeichnungen beispielhaft näher erläutert. Es zeigen:
- Figuren 1A, 1B:: eine erste Ausführungsform der erfindungsgemäßen Vorrichtung mit einem Fixateur externe in Streckstellung des Knies bzw. leichter Beugestellung,
- Figur 1C:: einen Fixateur externe mit einem Kraftelement zur Erzeugung einer nach vorne oder nach hinten gerichteten Translationskraft,
- Figuren 2A, 2B:: eine zweite Ausführungsform der erfindungsgemäßen Vorrichtung mit einer von außen an den Oberschenkel angelegten Orthese, wobei der Lastübertragungsarm auf einen Tibianagel einwirkt,
- Figuren 2C, 2D:: konstruktive Details eines Federgehäuses zur Erzeugung einer nach vorne oder nach hinten gerichteten Translationskraft
- Figuren 3A, 3B:: eine weitere Ausführungsform der erfindungsgemäßen Vorrichtung mit einer am Unterschenkel angelegten Unterschenkel-Orthese,
- Figuren 4A, 4B:: eine weitere Ausführungsform der erfindungsgemäßen Vorrichtung mit einem nach hinten drückenden Lastübertragungsarm zur Behandlung einer vorderen Instabilität, und
- Figuren 5A, 5B:: eine weitere Ausführungsform der erfindungsgemäßen Vorrichtung, mit welchem die Translationskraft ohne skelettäre Fixation aufgebracht werden kann.

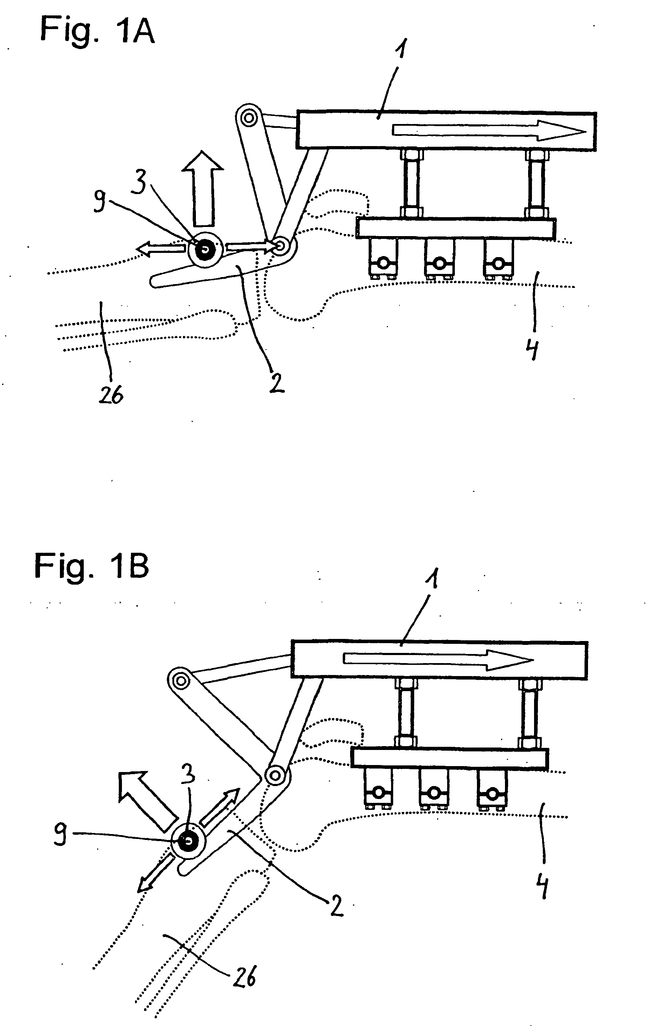

Aus den Figuren 1A, 1B ist ein Fixateur externe lateral ersichtlich, der mit mindestens zwei Schanzschrauben femoral unilateral fixiert ist und über zwei rechtwinklige Gestänge Kräfte nach ventral auf die Vorderseite des Oberschenkels und des Kniegelenkes zu einem rohrförmigen runden oder kantigen Apparat 1 übertragen kann. Der Apparat 1 enthält eine Druckfeder, die über einen beidseitig des Kniegelenkes herabgeführten Lastübertragungsarm 2 in der Form eines Winkelhebers eine Kraft einstellbarer Größe auf die Tibia überträgt.

Quer durch die Tibia, auf Höhe der Tuberositas tibiae wird ein Nagel, zweckmäßigerweise ein Steinmannnagel, gebohrt, auf dessen beiden Enden eine bewegliche Rolle 3 läuft, in denen die beidseitigen Winkelheber zur Ausübung der nach vorne gerichteten Kraft angreifen. Durch ventralen Druck, beispielsweise von 60 N in Streckstellung, kann das Kniegelenk auf 50° flektiert werden (mehr Beweglichkeit wird durch die Verankerungsnägel im Femur 4, die die Oberschenkel-Muskulatur durchqueren, verhindert), wobei gleichzeitig die nach ventral gerichtete Kraft auf 120 N zunimmt. Somit kann dem zunehmenden dorsal gerichteten Zug der ischiokruralen Muskulatur entgegengewirkt werden.

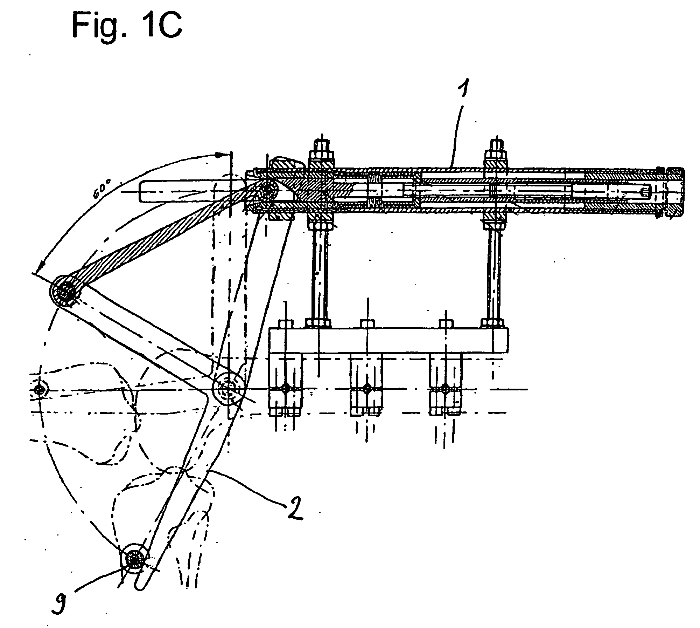

Figur 1C zeigt eine Ausführungsform eines Fixateur externe mit einer beispielhaften Ausführungsform eines Kraftelementes zur Erzeugung einer nach vorne oder nach hinten gerichteten Translationskraft.

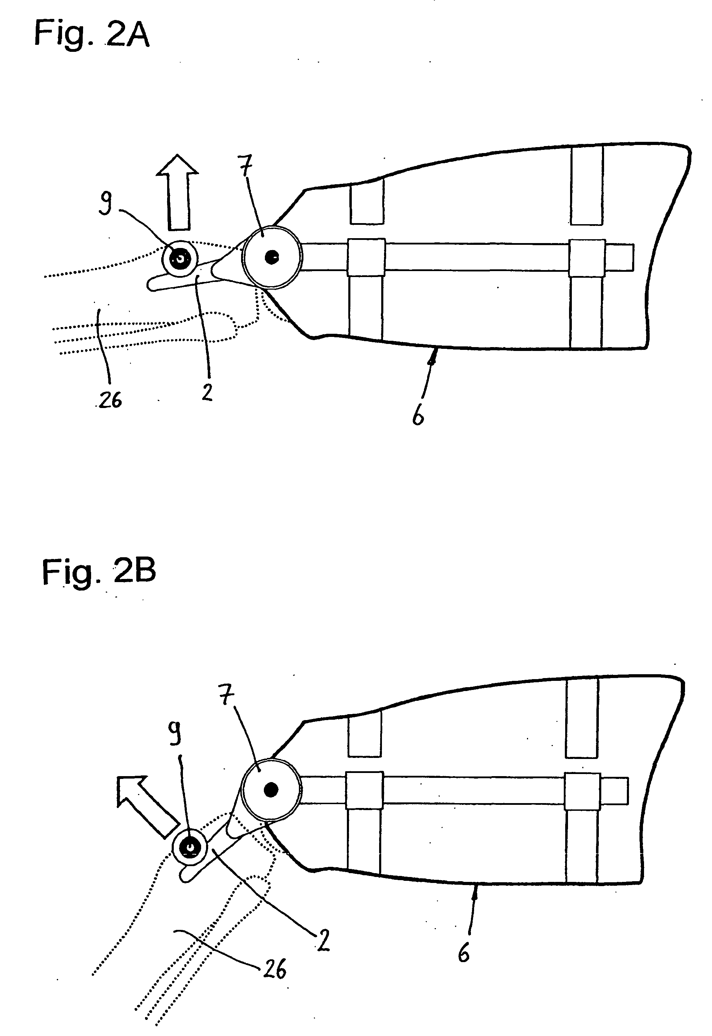

Die Figuren 2A, 2B zeigen ein Kraftelement zur Erzeugung einer nach vorne oder nach hinten gerichteten Translationskraft. Das Kraftelement ist mit einer Oberschenkelorthese 6 am Oberschenkel befestigt. Das Kraftübertragungssystem ist auf einem Kunststoffapparat befestigt, der nicht skelettär verankert, sondern außen am Oberschenkel fixiert wird. Durch Gipsabdruck und Kunststoff-Molding oder durch "off the shelf"-Technik wird ein "Brace", d.h. eine Orthese (Gelenkschiene) für den Oberschenkel und Unterschenkel angefertigt. Die Oberschenkelorthese 6, die beidseitig seitlich des Kniegelenks mit einem Federgehäuse 7 versehen ist, das eine regulierbare Feder 8 enthält, übt in dieser Anwendung ohne die Notwendigkeit einer skelettären Fixation, sondern durch eine rein äußere Applikation die gewünschte, nach vorne gerichtete Kraft auf einen Tibianagel 9 auf. Hier verteilt sich allerdings der Druck auf den muskulär-ossären Apparat und die entsprechenden äußeren Konturen im Kniebereich, was die Effizienz der Vorrichtung etwas reduziert, aber den Patientenkomfort verbessert.

Im Unterschied zur skelettären Fixation ist die dadurch ansetzende Kraft geringer, da sie weniger direkt ist. Die dabei ermöglichte Kniebeweglichkeit nimmt allerdings auf 90° zu.

Die beiden Federgehäuse 7 können seitlich ausgetauscht werden, wenn nicht eine dynamische vordere, sondern hintere Translation gewünscht ist.

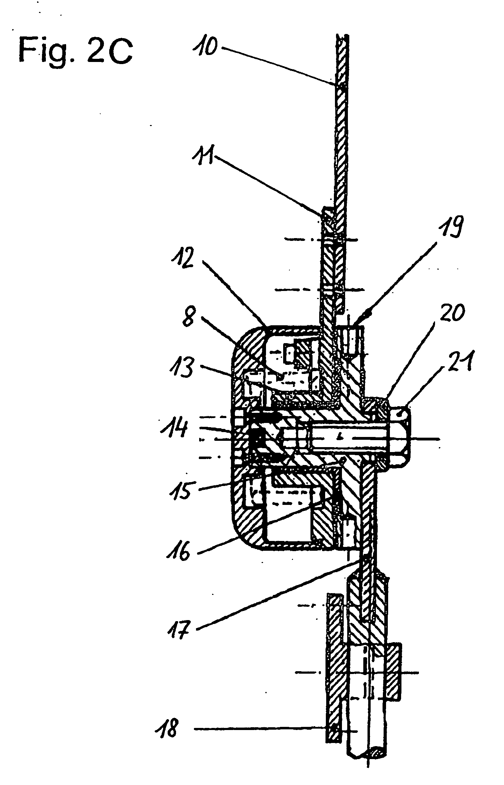

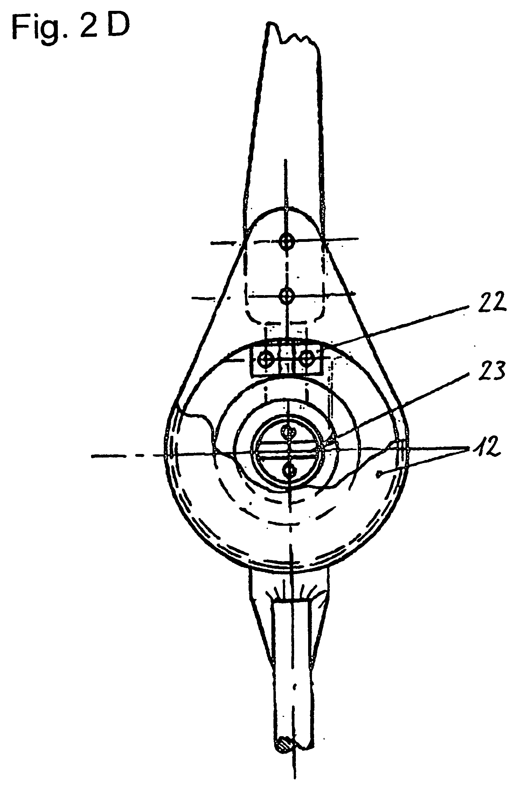

Die Figuren 2C, 2D zeigen konstruktive Details eines Federgehäuses 7 zur Erzeugung einer nach vorne oder nach hinten gerichteten Translationskraft. Hierbei zeigt Figur 2C einen Hebeleinsatz 10, einen Spannhebel 11, eine Skalahaube 12, die Feder 8, einen Spannhebel 13, einen Federhaken 14, ein Spannrad 15, eine Schleifbüchse 16, einen Halter 17, ein Fixierstück 18, Spannlöcher 19, eine Spannscheibe 20 und eine Spannschraube 21. Aus Figur 2D ist eine Federbride 22, ein Halteschlitz 23 und die Skalahaube 12 erkennbar.

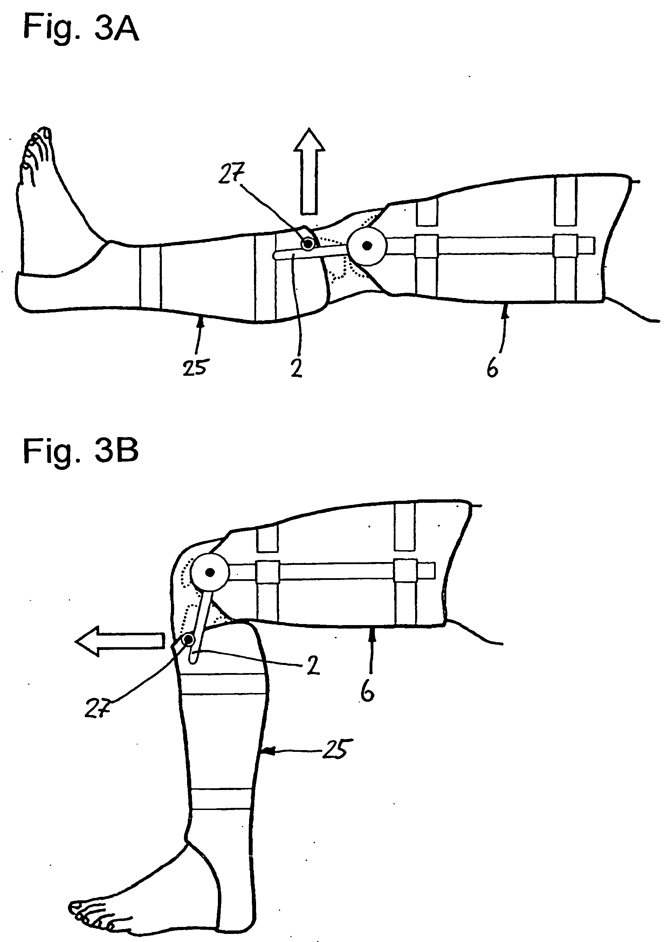

Wie aus den Figuren 3A, 3B ersichtlich, kann, falls sich der Tibianagel 9 nach zwei Monaten lockert oder falls man von Anfang an auf jegliche direkte skelettäre Fixation verzichten will, die Kraftübertragung auf die Tibia über eine am Unterschenkel angelegte Unterschenkelorthese 25 erzeugen, die zur Übernahme der Kraft auf entsprechender Höhe eine beidseitige stiftförmige Vorrichtung trägt, hinter der der Lastübertragungsarm 2 der Oberschenkelorthese 6 ansetzt und somit die nach vorne gerichtete Kraft ausübt, wodurch sich eine Translation der Tibia nach vorne ergibt. Bei der stiftförmigen Vorrichtung handelt es sich um Bolzen 27.

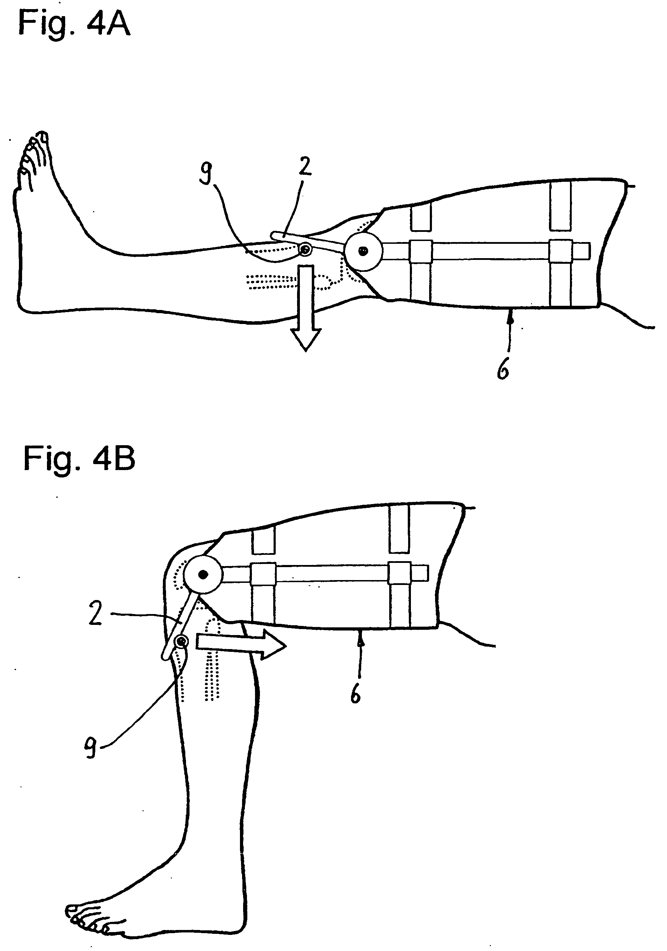

Wie aus den Figuren 4A, 4B ersichtlich, wird dann, wenn man zur Behandlung der vorderen Instabilität eine umgekehrte, hintere Translation wünscht, das Kraftelement 5 umgekehrt am Fixateur externe eingesetzt, so dass der Lastübertragungsarm 2 nach hinten drückt.

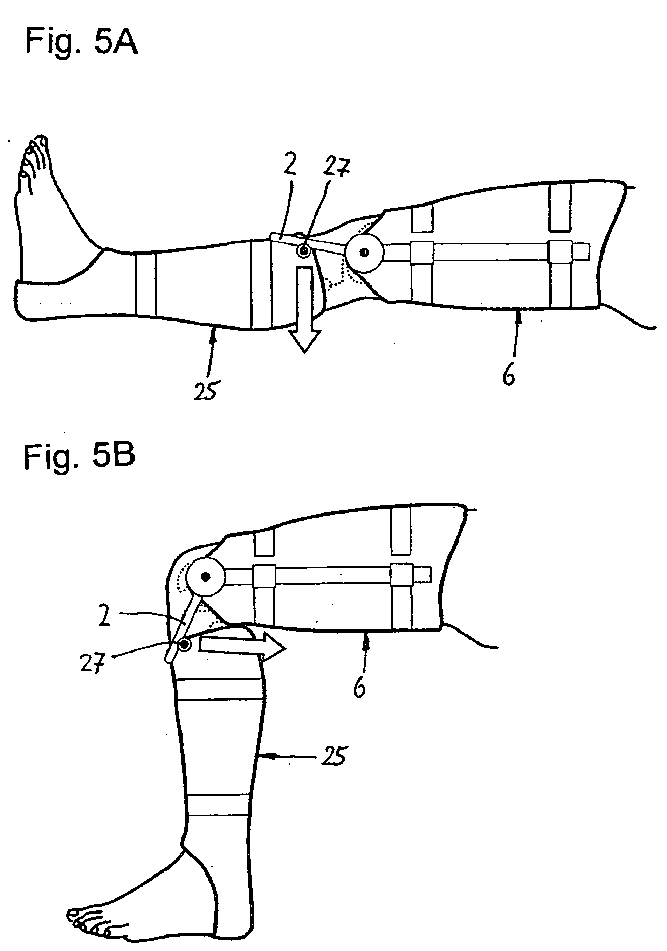

Die Figuren 5A, 5B zeigen eine Ausführungsform einer Unterschenkelorthese 25, mit welcher die Translationskraft ohne skelettäre Fixation aufgebracht werden kann. Dieses vollständig "skelettfreie" Fixationssystem kann ungestört über eine Zeitspanne von 4-5 Monaten getragen werden, während das am Skelett direkt fixierte System nach 2 Monaten Probleme des Patientenkomforts, der möglichen Pintrack Infektion und der limitierten Beweglichkeit mit sich bringt.

Bei allen Anwendungen liegt der wesentliche Punkt darin, dass eine starre femorotibiale Verbindung im Sinne einer konventionellen adynamischen oder dynamischen Schiene mit fixem Rotationszentrum und fixer Verbindung zwischen dem oberen Femurteil und dem unteren Tibiateil vermieden wird. Bei den Ausführungsformen gemäß den Figuren 1A bis 3B wird eine nach vorne gerichtete Kraft ungeachtet von der Lage der Vorrichtung bezüglich des Rotationszentrums ausgeübt, wodurch eine Interferenz des Systems mit der Geometrie oder Gelenkskonturen und dem physiologischen Roll-Gleitmechanismus des Kniegelenks verhindert wird. Damit ist die vordere Translationskraft unabhängig von der Flexionsstellung anwendbar.

Der Fixateur externe bzw. die Oberschenkelorthese 6 kann über den einseitig oder beidseitig des Kniegelenkes herabgeführten Lastübertragungsarm 2 eine - ungeachtet der jeweiligen Flexionsstellung des Kniegelenkes - nach vorne gerichtete Kraft einstellbarer Größe innen und außen auf den quer in die Tibia eingebrachten, mit Rollen 3 oder einem Gleitmechanismus versehenen Steinmannnagel oder auf die außen anlegbare, mit seitlichen Bolzen 27 versehene Unterschenkelorthese 25 ausüben, wobei die Bolzen 27 Rollen 3 oder einen Gleitmechanismus aufweisen.

Weiterhin ist es möglich, dass der Steinmannnagel oder das sonstige skelettär oder auf einer Orthese befestigte mechanische Element derart ausgebildet ist, dass er bzw. es eine zusätzliche Seit-zu-Seit-Verschiebung zulässt, wobei die physiologische Kinematik des Kniegelenkes, insbesondere Rotation und Schlussrotation, unbeeinträchtigt gelassen wird.

## Patentansprüche

1. Vorrichtung zur Ausübung einer Translationskraft auf die Tibia für die Behandlung oder Nachbehandlung der hinteren oder vorderen Kreuzbandinstabilität und assoziierter peripherer Knieinstabilitäten, welche einen Lastübertragungsarm (2) und zwei im Bereich des Kniegelenks befestigbare Teile aufweist, die über den Lastübertragungsarm (2) ohne starre femorotibiale Verbindung miteinander wirkverbunden sind, um die Translationskraft vom Femur (4) auf die Tibia (26) zu übertragen, wobei die Translationskraft in ihrer Größe einstellbar ist, **dadurch gekennzeichnet, dass** eines der Teile aus einem Fixateur externe besteht, welcher am Femur (4) fixierbar ist und ein eine statische oder dynamische Translationskraft erzeugendes Kraftelement trägt, und das andere Teil ein mit Rollen (3) oder Gleitmechanismen versehenes, quer in die Tibia (26) einbringbares mechanisches Element oder mit Rollen (3) oder Gleitmechanismen versehene, seitlich an einer Unterschenkelorthese (25) befestigbare Bolzen (27) umfasst, und dass der Lastübertragungsarm (2) ohne starre Verbindung mit dem mechanischen Element oder den Bolzen (27) über die am Element bzw. den Bolzen (27) angeordneten Rollen (3) oder Gleitmechanismen in Wirkverbindung steht, um die Translationskraft nach vorne oder hinten auf die Tibia (26) zu übertragen.

2. Vorrichtung zur Ausübung einer Translationskraft auf die Tibia für die Behandlung oder Nachbehandlung der hinteren oder vorderen Kreuzbandinstabilität und assoziierter peripherer Knieinstabilitäten, welche einen Lastübertragungsarm (2) und zwei im Bereich des Kniegelenks befestigbare Teile aufweist, die über den Lastübertragungsarm (2) ohne starre femorotibiale Verbindung miteinander wirkverbunden sind, um die Translationskraft vom Femur (4) auf die Tibia (26) zu übertragen, wobei die Translationskraft in ihrer Größe einstellbar ist und eines der Teile aus einer äußerlich anlegbaren Oberschenkelorthese (6) besteht, **dadurch gekennzeichnet, dass** an der Oberschenkelorthese (6) ein eine statische oder dynamische Translationskraft erzeugendes Kraftelement befestigt ist und das andere Teil ein mit Rollen (3) oder Gleitmechanismen versehenes, quer in die Tibia (26) einbringbares mechanisches Element oder mit Rollen (3) oder Gleitmechanismen versehene, seitlich an einer Unterschenkelorthese (25) befestigbare Bolzen (27) umfasst, und dass der Lastübertragungsarm (2) ohne starre Verbindung mit dem mechanischen Element oder den Bolzen (27) über die am Element bzw. den Bolzen (27) angeordneten Rollen (3) oder Gleitmechanismen in Wirkverbindung steht, um die Translationskraft nach vorne oder hinten auf die Tibia (26) zu übertragen.

3. Vorrichtung nach Anspruch 1, **dadurch gekennzeichnet, dass** der Fixateur externe mit mindestens zwei Schrauben in der Form von Schanzschrauben am Femur (4) fixierbar ist.

4. Vorrichtung nach einem der vorhergehenden Ansprüche, **dadurch gekennzeichnet, dass** das in der Tibia (26) eingebrachte mechanische Element aus einem Steinmannnagel besteht.

5. Vorrichtung nach einem der vorhergehenden Ansprüche, **dadurch gekennzeichnet, dass** der Lastübertragungsarm (2) einseitig oder beidseitig des Kniegelenks herabgeführt ist.

6. Vorrichtung nach einem der vorhergehenden Ansprüche, **dadurch gekennzeichnet, dass** das mindestens eine Kraftelement in einer oder mehreren Ebenen starr fixierbar ist.

7. Vorrichtung nach einem der vorhergehenden Ansprüche, **dadurch gekennzeichnet, dass** das Kraftelement eine Spiralfeder, welche in Richtung ihrer Längsachse auf Zug oder Druck oder quer zu ihrer Längsachse beansprucht ist, ein System von Blattfedern, eine Uhrenfeder, einen Torsionsstab, ein pneumatisches, hydraulisches, osmotisches, elektromagnetisches, magnetisches, Bimetall- oder Memory-Element umfasst.

8. Vorrichtung nach einem der vorhergehenden Ansprüche, **dadurch gekennzeichet,** dass die Translationskraft in Streckstellung in ihrer Größe einstellbar ist.

9. Vorrichtung nach einem der vorhergehenden Ansprüche, **dadurch gekennzeichnet, dass** die Translationskraft bei nach vorne gerichteter Translationskraft mit zunehmender Beugung zunimmt.

## Claims

1. Device for exerting a translational force on the tibia for treatment or follow-up treatment of posterior or anterior cruciate ligament instability and of associated peripheral instabilities of the knee, which device comprises a load transmission arm (2) and two parts which can be secured in the area of the knee joint and which are operatively connected to one another via the load transmission arm (2) without rigid femorotibial connection, so as to transmit the translational force from the femur (4) to the tibia (26), the magnitude of the translational force being adjustable, **characterized in that** one of the parts consists of an external fixator which can be fixed on the femur (4) and carries a force element generating a static or dynamic translational force, and the other part comprises a mechanical element which is provided with rollers (3) or slide mechanisms and can be introduced transversely into the tibia (26) or comprises bolts (27) which are provided with rollers (3) or slide mechanisms and can be secured laterally on a below-knee orthosis (25), and **in that** the load transmission arm (2) is operatively connected, without rigid connection, to the mechanical element or the bolts (27) via the rollers (3) or slide mechanisms arranged on the element or bolts (27), so as to transmit the translational force forwards or rearwards onto the tibia (26).

2. Device for exerting a translational force on the tibia for treatment or follow-up treatment of posterior or anterior cruciate ligament instability and of associated peripheral instabilities of the knee, which device comprises a load transmission arm (2) and two parts which can be secured in the area of the knee joint and which are operatively connected to one another via the load transmission arm (2) without rigid femorotibial connection, so as to transmit the translational force from the femur (4) to the tibia (26), the magnitude of the translational force being adjustable and one of the parts consisting of an externally applicable above-knee orthosis (6), **characterized in that** a force element generating a static or dynamic translational force is secured on the above-knee orthosis (6), and the other part comprises a mechanical element which is provided with rollers (3) or slide mechanisms and can be introduced transversely into the tibia (26) or comprises bolts (27) which are provided with rollers (3) or slide mechanisms and can be secured laterally on a below-knee orthosis (25), and **in that** the load transmission arm (2) is operatively connected, without rigid connection, to the mechanical element or the bolts (27) via the rollers (3) or slide mechanisms arranged on the element or bolts (27), so as to transmit the translational force forwards or rearwards onto the tibia (26).

3. Device according to Claim 1, **characterized in that** the external fixator can be fixed on the femur (4) with at least two screws in the form of Schanz screws.

4. Device according to one of the preceding claims, **characterized in that** the mechanical element introduced into the tibia (26) consists of a Steinmann pin.

5. Device according to one of the preceding claims, **characterized in that** the load transmission arm (2) is guided down along one side or both sides of the knee joint.

6. Device according to one of the preceding claims, **characterized in that** the at least one force element can be fixed rigidly in one or more planes.

7. Device according to one of the preceding claims, **characterized in that** the force element comprises a helical spring, which is tensioned or compressed in the direction of its longitudinal axis or tensioned transversely to its longitudinal axis, a system of leaf springs, a watch spring, a torsion rod, a pneumatic, hydraulic, osmotic, electromagnetic, magnetic, bimetal or shape-memory element.

8. Device according to one of the preceding claims, **characterized in that** the translational force can be adjusted in its magnitude in the extension position.

9. Device according to one of the preceding claims, **characterized in that**, in the case of forwardly directed translational force, the translational force increases with increasing flexion.

## Revendications

1. Dispositif pour exercer sur le tibia une force de translation en vue d'un traitement ou d'un post-traitement contre l'instabilité du ligament croisé postérieur ou antérieur et contre les instabilités périphériques associées du genou, qui comprend un bras de transmission de charge (2) et deux parties susceptibles d'être fixées dans la zone de l'articulation du genou qui sont reliées l'une à l'autre en termes d'action via le bras de transmission de charge (2) sans liaison fémoro-tibiale rigide, afin de transmettre la force de translation du fémur (4) au tibia (26), la force de translation étant réglable vis-à-vis de sa taille, **caractérisé en ce que** l'une des parties est constituée par un fixateur externe qui est susceptible d'être fixé au fémur (4) et qui porte un élément de force générant une force de translation statique ou dynamique, et **en ce que** l'autre partie comprend un élément mécanique pourvu de galets (3) ou de mécanismes de coulissement et susceptible d'être introduit transversalement dans le tibia (26), ou bien elle comprend des goujons (27) pourvus de galets (3) ou de mécanismes de coulissement et susceptibles d'être fixés latéralement sur une orthèse de jambe (25), et **en ce que** le bras de transmission de charge (2) est en liaison d'action avec l'élément mécanique ou avec les goujons (27), sans liaison rigide, via les galets (3) ou via les mécanismes de coulissement agencés sur l'élément ou sur les goujons (27), afin de transmettre la force de translation au tibia (26) vers l'avant ou vers l'arrière.

2. Dispositif pour exercer sur le tibia une force de translation en vue du traitement ou du post-traitement contre l'instabilité du ligament croisé postérieur ou antérieur et contre les instabilités périphériques associées du genou, qui comprend un bras de transmission de charge (2) et deux parties susceptibles d'être fixées dans la zone de l'articulation du genou qui sont reliées l'une à l'autre en termes d'action via le bras de transmission de charge (2) sans liaison fémoro-tibiale rigide, afin de transmettre la force de translation du fémur (4) au tibia (26), la force de translation étant réglable vis-à-vis de sa taille et l'une des parties étant constituée par une orthèse de cuisse (6) à appliquer extérieurement, **caractérisé en ce qu'**un élément de force générant une force de translation statique ou dynamique est fixé sur l'orthèse de cuisse (6), et **en ce que** l'autre partie comprend un élément mécanique pourvu de galets (3) ou de mécanismes de coulissement et susceptible d'être introduit transversalement dans le tibia (26), ou bien elle comprend des goujons (27) pourvus de galets (3) ou de mécanismes de coulissement et susceptibles d'être fixés latéralement sur une orthèse de jambe (25), et **en ce que** le bras de transmission de charge (2) est en liaison d'action avec l'élément mécanique ou avec les goujons (27), sans liaison rigide, via les galets (3) ou via les mécanismes de coulissement agencés sur l'élément ou sur les goujons (27), afin de transmettre la force de translation au tibia (26) vers l'avant ou vers l'arrière.

3. Dispositif selon la revendication 1, **caractérisé en ce que** le fixateur externe est susceptible d'être fixé au fémur (4) par au moins deux vis sous la forme de vis de Schanz.

4. Dispositif selon l'une des revendications précédentes, **caractérisé en ce que** l'élément mécanique introduit dans le tibia (26) est constitué par un clou de Steinmann.

5. Dispositif selon l'une des revendications précédentes, **caractérisé en ce que** le bras de transmission de charge (2) descend d'un côté ou des deux côtés de l'articulation de genou.

6. Dispositif selon l'une des revendications précédentes, **caractérisé en ce que** ledit au moins un élément de force est susceptible d'être fixé rigidement dans un ou dans plusieurs plans.

7. Dispositif selon l'une des revendications précédentes, **caractérisé en ce que** l'élément de force comprend un ressort spiralé qui est sollicité en traction ou en compression dans la direction de son axe longitudinal ou transversalement à son axe longitudinal, un système de ressorts à lames, un ressort de montre, une barre de torsion, un élément pneumatique, hydraulique, osmotique, électromagnétique, magnétique, bilame ou à effet mémoire.

8. Dispositif selon l'une des revendications précédentes, **caractérisé en ce que** la force de translation est réglable vis-à-vis de sa taille en position étirée.

9. Dispositif selon l'une des revendications précédentes, **caractérisé en ce que**, lorsque la force de translation est dirigée vers l'avant, celle-ci augmente au fur et à mesure de la flexion.
